# EUROPEAN PATENT SPECIFICATION

(11) **EP 4 370 595 B1**
(45) Date of publication and mention of the grant of the patent: **03.09.2025**
(21) Application number: 22748046.4
(22) Date of filing: 13.07.2022
(51) Int. Cl.: C08J 11/24, C08G 18/82, C07C 265/12, C07C 271/06, C07C 263/04

(54) **TWO-STAGE CHEMICAL RECYCLING OF POLYURETHANES**
ZWEISTUFIGES CHEMISCHES RECYCLING VON POLYURETHANEN
RECYCLAGE CHIMIQUE DE POLYURÉTHANES EN DEUX ÉTAPES

(30) Priority: 14.07.2021 EP 21185601
(43) Date of publication of application: 22.05.2024
(73) Proprietor: Universiteit Antwerpen, 2000 Antwerpen (BE)
(72) Inventor: BILLEN, Pieter, 2000 Antwerpen (BE); PAZDUR, Lukasz, 2000 Antwerpen (BE); VANDE VELDE, Christophe, 2000 Antwerpen (BE)
(74) Representative: Arnold & Siedsma
(86) International application number: PCT/EP2022/069629
(87) International publication number: WO 2023/285545

(56) References cited:
- EP-A1- 2 213 658
- WO-A1-2019/219814
- WO-A1-2020/260387
- WO-A1-2021/023889
- WO-A1-2022/263336
- WO-A1-95/24384
- KR-A- 20090 089 211

## Description

### FIELD OF THE INVENTION

The present invention relates to the field of polyurethane recycling and pertains to a method of recycling of a polyurethane, comprising a first alcoholysis step and a further thermolysis step. By means of the method according to the present invention, it is possible to recover both the polyols and isocyanates from said polyurethane.

### BACKGROUND TO THE INVENTION

Polyurethanes are very versatile polymers being used as binders, foams (rigid and flexible), adhesives and sealants, elastomers, coatings and binders, among others. Given the diversity of applications, in mattresses, upholstery, dashboard liners, adhesives, sealants, etc., global production rates are increasing, to currently about 18 million tonnes annually. Therefore, the demand for polyurethanes and their precursors is high, and their production processes, especially the phosgene process, are under more and more scrutiny. Companies involved in producing and using polyurethanes, as well as governing agencies, are increasingly willing to move beyond recycling methodology in the state of the art, towards better recycling methods.

Polyurethanes are made by reacting an isocyanate, such as toluene diisocyanate (TDI) or methylene diphenyl diisocyanate (MDI), with a long chain dialcohol prepolymer, called a polyol. In the state of the art, the recycling of products made of polyurethanes is accomplished mainly by means of two approaches. A first approach involves a glycolysis reaction (or other alcoholysis) of the polyurethanes, which allows recovery of a polyol fraction and a dicarbamate (or diurethane) fraction, of e.g. the original diisocyanate monomer and glycol (or other alcohol) used to synthesize the polyurethane. Optionally, starting from this first approach, the dicarbamate fraction is hydrolyzed, providing chemically unstable dicarbamic acid, which shortly decomposes into diamine and CO₂. The diamine obtained is of inferior value, as it has to undergo energy-intensive work-up to a diisocyanate, often involving unwanted toxic chemicals. A second recycling approach involves the direct pyrolysis of the polyurethanes, which yields a wide variety of products, as the high temperature degrades the polyols as well as the urethane (carbamate) linkages. Approaches of recycling of polyurethanes have shortcomings which overall balance out the advantages given by the recycling process itself, making the recycling of polyurethanes time and resource consuming, not environmentally friendly and poorly economically viable.

WO20260387A1 discloses a method of recycling of polyurethane products involving an alcoholysis reaction and a phase separation process leading to the obtainment of a polyol fraction derived from the polyurethanes and amines, which are obtained from the hydrolysis of the carbamates. A disadvantage of WO20260387A1 is that the produced amines are not merely as reactive as isocyanates, and to close the material cycle and produce new polyurethane products from the recovered amines, said amines need to be transformed to more reactive equivalents. In particular, they might have to be subjected to either a costly and environmentally undesirable phosgene synthesis process leading to isocyanates, or an energy-intensive phosgene-free process leading an isocyanate equivalent.

KR 20090089211 A discloses a method for manufacturing polyisocyanate from polyurethanes scraps, wherein the method comprises a step of reacting the polyurethane scraps with the glycol or amine, and heating the scraps to obtain a depolymerized material; removing polyols and glycol from the depolymerized material, and reacting an amine compound, which is the rest intermediate product with a dicarbonate compound to obtain carbamate; and synthesizing isocyanate from carbamate using a silane compound catalyst, wherein the reaction is carried out at a temperature of 70 °C.

WO 95/24384 A1 discloses a process for preparing a polyisocyanale, comprising pyrolyzing a polycarbamate, to form the corresponding polyisocyanate and alcohol, wherein the process is carried out at temperatures from 100°C and 250°C, in the absence of solvent and a catalyst.

WO 2021/023889 A1 discloses a method for alcoholysing and hydrolysing a polyurethane (PUR) materials, made from at least one polyol compound and at least one toluene diisocyanate based compound, wherein the method comprises a step of reacting the polyurethane material with at least one alcoholizing compound, thereby forming a mixture and a step of allowing the mixture to separate into at least a lower phase, which comprises at least a toluene diamine compound and a toluene dicarbamate and an upper phase, which comprises at least one recovered polyol from which the PUR material was made.

WO 2019/219814 discloses a method for alcoholysing a polyurethane (PUR) material, made from at least one polyol compound having a hydroxyl value X and at least one polyisocyanate compound, wherein the method comprises a step of reacting the polyurethane material with at least one alcoholysing compound, thereby forming a mixture, which is allowed to separate into at least two immiscible phase, and wherein the reaction mixture further comprises at least one alcoholysis accelerator, wherein the carbamates in the PUR material are converted into compounds comprising a primary and/or secondary amine.

EP 2 213 658 A1 discloses a process for producing a divalent aromatic hydroxy compound and an isocyanate compound, comprising the steps of reacting an aromatic polycarbonate resin and an amine compound having a primary amino group to obtain a mixture containing a carbamic acid ester and a compound having an aromatic hydroxyl group, which are originated from the aromatic polycarbonate; and subjecting the carbamic acid to a thermal decomposition reaction to obtain the divalent aromatic hydroxy compound and the isocyanate compound.

There is therefore the industrial need to provide a new recycling method of polyurethanes, overcoming the drawbacks in the prior art. The present invention aims at providing a more straightforward and environmentally friendly method of recycling of polyurethane products.

### SUMMARY OF THE INVENTION

In a first aspect, the present invention relates to a method of recycling of a polyurethane, comprising the steps of:
a) providing a polyurethane A;
b) providing a first alcohol B, either polyfunctional or monofunctional selected from the list comprising: propanol, pentanol, isopropyl alcohol, butanol, hexanol, nonanol, octanol;
c) performing an alcoholysis reaction of the polyurethane A provided at step a) with the first alcohol B provided at step b), thereby obtaining a carbamate C of said first alcohol B, and a polyol D,
d) separating the carbamate C from the polyol D, obtained at step c), thereby providing the carbamate C;
e) performing a thermolysis reaction on the carbamate provided at step d), thereby obtaining an isocyanate E and an alcohol F;
f) separating the isocyanate E from the alcohol F.

An advantage of the method according to the present invention is therefore its enabling full-cycle recycling. A further advantage of the present invention is its allowing obtainment of defined polyurethane constituents, i.e. an alcohol and an isocyanate from a polyurethane. Further, an advantage of the present invention is allowing the obtainment of an isocyanate from a polyurethane, not relying on e.g. phosgene utilization, for the synthesis of said isocyanates, thereby allowing a polyurethane recycling methodology that is more environmentally friendly and safer for manufacturing personnel compared to the state of the art. By means of the present invention, in a first step valuable polyols are safeguarded from thermolysis, whereas at a second step the thermal reversibility of carbamate bonds is exploited. The method according to the present invention thus overcomes the biggest disadvantages of the two aforementioned polyurethane recycling approaches part of the state of the art.

In a preferred embodiment according to the present invention, at step b) the first alcohol B is ethylene glycol. An advantage according to the present embodiment is that the use of ethylene glycol as first alcohol B provides for good separation results at step d), while having a sufficiently low vapor pressure on order to perform step c) at high temperature, while having a boiling point different from most isocyanate monomers to allow a separation in step f). By means of the present embodiment, phase separation in step d) can be preferably applied.

In a further embodiment according to the present invention, step d) further comprises the steps:
d2) providing an second alcohol G, either mono- or polyfunctional, different from the first alcohol B provided at step b);
d3) conducting a transcarbamation reaction on the carbamate C provided at step d), thereby obtaining a carbamate H of the second alcohol G provided at step d2), and the first alcohol B;
d4) separating the carbamate H obtained at step d3) from the first alcohol B.

An advantage of the present embodiment is that it allows the fine-tuning of the stability of the carbamates to be thermolyzed and hence the kinetics of this reaction, and of the separation after alcoholysis and thermolysis, and thus to select the best thermolysis candidates. For example, the first alcohol B can be carefully selected based on the ease of separation of the carbamate C obtained therefrom at step d), without being disadvantageous to the thermolysis reaction. By means of the transcarbamation reaction, further optimization of the separation post thermolysis, can be achieved as it allows for the replacement of said first alcohol B with a second alcohol G, enabling fine-tuning of the final separation step while recovering the first alcohol B. Second alcohol G may provide lower thermal stability of the carbamate bond, and/or may have a bigger boiling point difference from the isocyanate E.

In a further embodiment according to the present invention, at step e) the thermolysis reaction is performed at a temperature from about 200 ºC to about 450 ºC, preferably from about 200 ºC to about 300 ºC. It has been found that the present embodiment provides for the best thermolysis conditions, minimizing the occurrence of side-reactions other than the thermolysis of the carbamate and providing less side-products such as oligomers, polymers and carbodiimides.

In a further embodiment according to the present invention, at step a) the polyurethane A, includes mixtures of polyurethanes, i.e. comprising at least two polyurethanes. An advantage of the present embodiment is that the recycling can be performed on larger amounts of materials, without the need for prior separation of the polyurethanes by type.

In a further embodiment according to the present invention, at step f) the isocyanate E is separated from the alcohol F by distilling away the alcohol F at a temperature from about 100 ºC to about 300 ºC, preferably from about 200 ºC to about 250 ºC, more preferably from 210 ºC to about 230 ºC.

In a further embodiment according to the present invention, at step c) the alcoholysis reaction is performed at a temperature from about 120 ºC to about 240 ºC, preferably from about 150 ºC to about 200 ºC, more preferably from 180 ºC to about 190 ºC. The alcoholysis reaction should be done at the maximum temperature at which evaporation of the alcohol B is acceptably low and at which the thermal degradation of the reactants is avoided, while at the same time ensuring sufficiently fast reaction kinetics.

In a further embodiment according to the present invention, at step d2) the the second alcohol G is a C₁-C₁₅ alcohol, preferably a C₁-C₁₀ alcohol, more preferably a C₄-C₈ alcohol. Alcohol B should preferably have a high boiling point and a polarity different from the polyols used to enable the formation of at least two separate phases in step c) and to facilitate easy separation in step d). Alcohol G should preferably have a boiling point and polarity significantly different from the isocyanate E, to facilitate the separation in step f), while leading to a moderately low thermal stability of the carbamate bond in carbamate C or H.

In a further embodiment according to the present invention at step b) the first alcohol B is polyfunctional and selected from the list comprising glycerol, ethylene glycol, diethylene glycol, triethylene glycol. In a further embodiment according to the present invention, at step d2) the second alcohol G is selected from the list comprising: propanol, pentanol, isopropyl alcohol, butanol, hexanol, nonanol, octanol, glycerol, ethylene glycol, diethylene glycol, triethylene glycol.

In a further embodiment according to the present invention, at step b) and/or d2) the first alcohol B and/or the second alcohol G is provided in excess, wherein the molar excess of said first alcohol B and/or second alcohol G respect to the polyol D is of at least 2:1, preferably exceeding 3:1, more preferably exceeding 4:1.

In a further embodiment according to the present invention, at step e) the thermolysis reaction is performed at a pressure from about 1 bar (100 kPa) to about 100 mbar (10 kPa), preferably from about 1 bar (100 kPa) to about 200 mbar (20 kPa), J more preferably from 1 bar (100 kPa) to about 0.9 bar (90 kPa); or under an inert gas. J A reduced pressure compared to atmospheric pressure leads to lower required operating temperatures, but may induce higher operational costs.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings is provided to make apparent to those skilled in the art how the several forms of the invention may be embodied in practice.
**Figure 1****,** also referred to as **Fig. 1****,** illustrates the method of recycling according to the present invention.
**Figure 2****,** also abbreviated as **Fig. 2****,** illustrates an embodiment according to the present invention, wherein a carbamate (obtained after alcoholysis of the polyurethane) is subjected to a transcarbamation reaction prior to thermolysis.
**Figure 3****,** also abbreviated as **Fig. 3****,** exemplifies how the method of recycling according to the present invention can be put into practice. More specifically, **Fig. 3** illustrate how the method according to the present invention can be used to recycle a polyurethane foam comprising toluene diisocyanate (TDI) and polypropylene glycol (PPG) units.
**Figure 4****,** also abbreviated as **Fig. 4****,** illustrates a schematic overview of the performed thermolysis reactions for dicarbamates of the following alcohols: methanol, isopropanol, butanol, ethylene glycol and octanol.
**Figure 5****,** also abbreviated as **Fig. 5****,** illustrates results of TGA analysis (thermal gravimetric analysis) for ((Bis(2-hydroxyethyl)(methylenebis(1,4-phenylene))dicarbamate (EMd)). In the figure, the TGA curve is plotted starting from the top left (100% weight), whereas the other curve, represents the DTG curve (first derivative of the TGA curve).
**Figure 6****,** also abbreviated as **Fig. 6****,** illustrates results of SEC chromatogram of the solid OMd thermolysis mixture after 0, 1, 5, 10 and 15 minutes for the thermolysis of octanol-based dicarbamates with carbamic acid,(methylenedi-1,4-phenylene)bis-, dioctyl ester (OMd), synthesized by a reaction of MDI with octanol.
**Figure 7****,** also abbreviated as **Fig. 7****,** illustrates results of SEC chromatogram of the solid BuMd thermolysis mixture after 5, 15 and 30 minutes for the thermolysis of dibutyl 4,4'-methylenediphenylenedicarbamate (BuMd).
**Figure 8****,** also abbreviated as **Fig. 8****,** illustrates results of SEC chromatogram of the solid IsoMd pyrolysis mixture after 5, 15 and 30 minutes for the thermolysis of Diisopropyl 4,4'-methylenediphenylenedicarbamate (IsoMd)
**Figure 9****,** also abbreviated as **Fig. 9****,** provides a schematic overview of the blocking reaction of the isocyanate functionalities present in the thermolysis reaction mixtures, prior to a quantitative HPLC analysis method.
**Figure 10****,** also abbreviated as **Fig. 10****,** provides the TOF-MS spectrum of the solid IsoMd pyrolysis mixture after 15 minutes and after the blocking reaction of the isocyanate groups.
**Figure 11****,** also abbreviated as **Fig. 11****,** provides the SEC chromatogram of a solvent assisted thermolysis reactions of IsoMd using silicon oil (upper line in the graph) and tetraethylene glycol dimethyl ether (lower line in the graph).

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. When describing the compounds of the invention, the terms used are to be construed in accordance with the following definitions, unless a context dictates otherwise.

The term "about" or "approximately" as used herein when referring to a measurable value such as a parameter, an amount, a temporal duration, and the like, is meant to encompass variations of +/- 10 % or less, preferably +/- 5 % or less, more preferably +/- 1 % or less, and still more preferably +/- 0.1 % or less of and from the specified value, insofar such variations are appropriate to perform in the disclosed invention. It is to be understood that the value to which the modifier "about" or "approximately" refers is itself also specifically, and preferably, disclosed.

In a first aspect, the present invention relates to a method 100 of recycling of a polyurethane. In the context of the present invention, by means of the term "recycling", reference is made to the action or process of converting waste, in this case a polyurethane product, in other words a product comprising at least a polyurethane, into reusable material, such as, and not limited to, polyols, amines, carbamates and isocyanate from said polyurethane. In the context of the present invention, recycling pertains more specifically to the act of recovering a polyol or polyol phase, and/or an isocyanate or isocyanate-rich phase, from a polyurethane. The present invention enables the full recycling of a polyurethane, meaning that a polyurethane can be potentially regenerated from the recovered polyol and isocyanate, by means of reacting the polyol and the isocyanate together. In these latter circumstances, the recycling is a closed-loop recycling, wherein a product or material comprising polyurethanes can be used and then converted back to raw materials. Nevertheless, either the recovered polyols or the recovered isocyanate can be also employed in other manufacturing processes, either for the production of new polyurethanes from polyols or isocyanates the initially recycled polyurethane was not based on, in the manufacturing of other products, or as raw chemicals per-se.

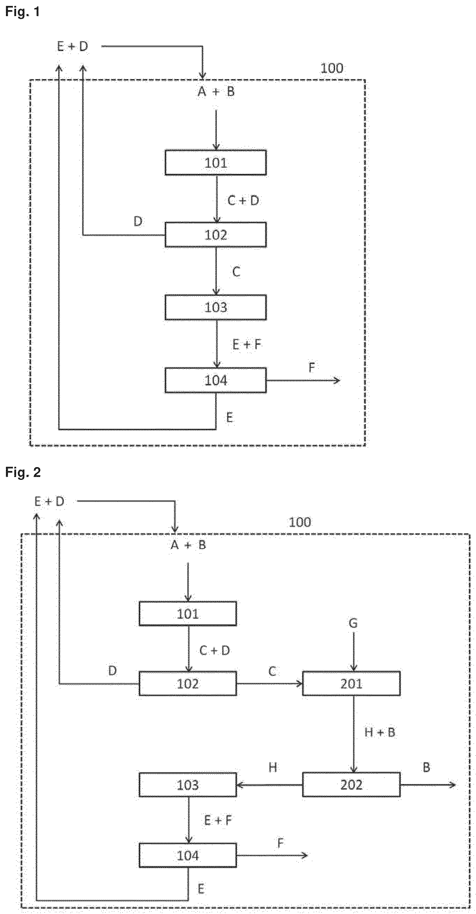

**Fig. 1** illustrates the method according to the present invention. The method 100 according to the present invention, of recycling of a polyurethane, comprises the steps here below, which are further explained in the present application:
a) providing a polyurethane A;
b) providing a first alcohol B, either mono- or polyfunctional
c) performing an alcoholysis reaction 101 of the polyurethane A provided at step a) with the first alcohol B provided at step b), thereby obtaining a carbamate C of said first alcohol B, and a polyol D,
d) separating 102 the carbamate C from the polyol D, obtained at step c), thereby providing the carbamate C;
e) performing a thermolysis reaction 103 on the carbamate provided at step d), thereby obtaining an isocyanate E and an alcohol F;
f) separating 104 the isocyanate E from the alcohol F.

As illustrated, the method 100 according to the present invention allows to recycle a polyurethane A, thereby providing at least one polyol D and an isocyanate E from said polyurethane A. The method according to the present invention allows for full-cycle recycling of the polyurethane A, as the isocyanate E and the polyol D can readily be reacted together to possibly form again polyurethane A, or other types of polyurethanes, by means of polymerization techniques known to the skilled in the art. An advantage of the method according to the present invention is therefore its enabling full-cycle recycling. A further advantage of the present invention is its allowing obtainment of defined polyurethane constituents, i.e. an alcohol and an isocyanate from a polyurethane. Further, an advantage of the present invention is allowing the obtainment of an isocyanate from a polyurethane, not relying on e.g. phosgene utilization, for the synthesis of said isocyanate, thereby allowing a polyurethane recycling methodology that is more environmentally friendly and safer for manufacturing personnel compared to the state of the art. By means of the present invention, in a first step valuable polyols are safeguarded from thermolysis, whereas at a second step the thermal reversibility of carbamate bonds is exploited. The method according to the present invention thus overcomes the biggest disadvantages of the two aforementioned polyurethane recycling approaches part of the state of the art, meaning a first approach wherein the dicarbamate fraction is hydrolyzed, and a second approach involving direct pyrolysis of the polyurethanes. This allows no need for conducting other reactions aiming at the formation of isocyanates via e.g. phosgene utilization, which would present environmental and safety risks associated thereto. In case the alcoholysis product is directly subjected to a thermolysis reaction after separation, the alcohol F can correspond to the first alcohol B. Possibly, the alcohol B separated at step 104 can also be recycled for the alcoholysis 101, thereby further improving the environmental footprint of the present invention.

**Fig. 2** further discloses an embodiment of the present invention. **Fig. 2** illustrates steps a) to f) according to the present invention, wherein the product carbamate C from the alcoholysis reaction 102 is further processed. In particular, the carbamate C is subjected to a transcarbamation reaction with a second alcohol G, different from the first alcohol B. After the transcarbamation reaction has taken place, a carbamate H is recovered. In particular, in accordance with the present embodiment, step d) further comprises:
d2) providing an second alcohol G, either mono- or polyfunctional, different from the first alcohol B provided at step b);
d3) conducting a transcarbamation reaction 201 on the carbamate C provided at step d), thereby obtaining the first alcohol B and a carbamate H of the second alcohol G provided at step d2), and;
d4) separating 202 the carbamate H obtained at step d3) from the first alcohol B.

Preferably, the first alcohol B separated at d4) can be further reutilized in the alcoholysis reaction method according to the present invention.

If need be, the carbamate H separated at step d4) can be further subjected to a transcarbamation reaction. An advantage of the present embodiment is that both the separation of the carbamate after alcoholysis and the thermolysis can be purposefully selected, and the overall method better fine-tuned for the obtainment of higher yields.

Therefore, the method 100 according to the present invention comprises a step a) of providing a polyurethane A. In the context of the present invention, by means of the term "polyurethane", also referenced with the acronyms "PU" or "PUR", reference is made to a polymer composed of organic units joined by carbamate bonds (-NH-(C=O)-O-), which link molecular units together. Polyurethanes are traditionally and most commonly formed by reacting an isocyanate (di- or triisocyanate) with a polyol. Polyurethanes suitable to be used in the context of the present invention can be thermoplastic or thermoset polyurethanes, whereby in the former no (or at least very limited) urea and biuret linkages are expected. Depending on the type of isocyanates and polyols utilized for the manufacturing of the polyurethanes, a multitude of material properties can be obtained, resulting in rigid foams, flexible or viscoelastic (memory) foams, or films. Polyurethanes are a diverse class of heteropolymers, made by reacting isocyanate with polyol monomers. Typical isocyanates that are used in the state of the art for the synthesis of polyurethanes are e.g. methylene diphenyl diisocyanate (MDI) and toluene diisocyanate (TDI), although higher functional isocyanates may be added to increase crosslinking and therefore stiffness in the final application. The polyols used are typically long-chain (ca. 10 000 g mol⁻¹) polyether or polyester polyols, with polyethylene glycol (PEG) or polypropylene glycol (PPG) as dominant examples. Branched, multifunctional long-chain polyols or short-chain polyols are sometimes used to increase crosslinking, or in the synthesis or rigid foams. Examples of polyurethanes which can be recycled in accordance with the present invention, include polyether flexible foams as well as rigid foams. The polyurethane provided at step a) can therefore be any kind of polyurethane polymer which can be recycled by means of the method of the present invention. For examples, polyurethanes that can potentially be recycled by means of the present invention include rigid and flexible polyurethanes. Both rigid and flexible polyurethanes can be successfully recycled by means of the present invention, as far as a polyol phase can be successfully separated from the carbamate containing phase.

In a further embodiment according to the present invention, at step a) the polyurethane A as used herein includes mixtures of polyurethanes comprising at least two different polyurethanes. Therefore, in accordance with the present embodiment, the polyurethane A provided a step a) can be a mixture of two or more different polyurethane polymers, i.e. polyurethanes with different polyether polyols and/or different isocyanate monomers as constituents. Preferably, the mixture of two or more different polyurethanes is a mixture of either only rigid polyurethanes or flexible polyurethanes.

Further, the present invention comprises the step b) of providing a first alcohol B, either polyfunctional or monofunctional selected from the list comprising: propanol, pentanol, isopropyl alcohol, butanol, hexanol, nonanol, octanol. In the context of the present invention, by means of the term "alcohol", either a first alcohol B, which is provided for the alcoholysis reaction, or a second alcohol G, which is provided for the transcarbamation reaction, reference is made to a compound comprising at least one alcohol moiety e.g. R-O-H. Therefore, the first alcohol provided in accordance with the present invention is either monofunctional, thereby comprising a single -O-H and selected from the list comprising: propanol, pentanol, isopropyl alcohol, butanol, hexanol, nonanol, octanol, or polyfunctional, comprising therefore 2 or more -O-H groups.

In a further embodiment according to the present invention, at step d2) the the second alcohol G is a C₁-C₁₅ alcohol, preferably a C₁-C₁₀ alcohol, more preferably a C₄-C₈ alcohol. In a further embodiment according to the present invention, at step d2) the second alcohol G is selected from the list comprising propanol, pentanol, isopropyl alcohol, butanol, hexanol, nonanol, octanol, glycerol, ethylene glycol, diethylene glycol, triethylene glycol. In a further embodiment according to the present invention, at step b) the first alcohol B is polyfunctional and it is selected from the list comprising ethylene glycol, diethylene glycol, triethylene glycol.

In a further embodiment according to the present invention, at step b) and/or d2) the first alcohol B and/or the second alcohol G is provided in excess, wherein the molar excess of said first alcohol B and/or second alcohol G with respect to the polyol D is at least 2:1, preferably exceeding 3:1, more preferably exceeding 4:1.

In a preferred embodiment according to the present invention, at step b) the first alcohol B is ethylene glycol. An advantage according to the present embodiment is that the use of ethylene glycol as first alcohol B provides good and demonstrated separation results at step d). By means of the present embodiment, phase separation can be applied. In a specific embodiment of the present invention, wherein TDI and/or MDI are used, the use of ethylene glycol is further advantageous. The higher polarity of ethylene glycol compared to TDI and MDI makes it insoluble in mineral oil, which can be used as a solvent in the thermolysis to facilitate immediate phase separation in step e), thereby further providing ease of separation.

Further, the method according to the present invention comprises the step c) of performing an alcoholysis reaction 101 of the polyurethane A provided at step a) with the first alcohol B provided at step b), thereby obtaining a carbamate C of said first alcohol B, and a polyol D.

In the context of the present invention, by means of the term "alcoholysis", reference is made to a reaction wherein an alcohol moiety of an ester or carbamate e.g. -O-R in an ester R'-(C=O)-O-R is displaced by a different alcohol moiety e.g. -O-R". In the particular context of the present invention, when the alcoholysis involves polyurethanes, having e.g. formula -R-NH-(C=O)-O-R-O-, the alcohol moiety -O-R-O- is e.g. displaced by reaction with a different alcohol, such as R"-O-H, providing -R-NH-(C=O)-O-R". In case the alcohol utilized in the alcoholysis reaction is a glycol, such as ethylene glycol or diethylene glycol, the alcoholysis also goes by the name of glycolysis.

In a further embodiment according to the present invention, at step c) the alcoholysis reaction is performed at a temperature equal or lower than the boiling point of alcohol B.

In a further embodiment according to the present invention, at step c) the alcoholysis reaction is performed at a temperature from about 120 ºC to about 240 ºC, preferably from about 150 ºC to about 200 ºC, more preferably from 180 ºC to about 190 ºC.

In the context of the present invention, by means of the term "polyol", reference is made to a compound comprising at least two alcohol moieties e.g. R-(OH)ₙ, with n>=2, such as a diol, a triol etc..

In the context of the present invention, by means of the term "carbamate", reference is made to a compound a compound comprising a non-polymeric compound comprising at least a -NH-(C=O)-O- moiety, such as a monocarbamate, having general formula R-NH-(C=O)-O-R", a dicarbamate R‴-O-(C=O)-NH-R-NH-(C=O)-O-R", tricarbamate and so on. Therefore, in accordance with the present invention, and being the reaction product of the alcoholysis reaction of the polyurethane A provided in step a),the carbamate can be mono- di- or multi-, depending on the polyurethanes used provided at step a).

Further, the method according to the present invention comprises the step d) of separating 102 the carbamate C from the polyol D, obtained at step c), thereby providing the carbamate C.

The carbamate C can be separated from the polyol D by means of various separation techniques, such as, and not limited to, phase separation, filtration, distillation, chromatography, decantation etc. Preferably, when at step b) the first alcohol B is a glycol, such as ethylene glycol, the carbamate C can be separated from the polyol D by means of phase separation. In order to provide for the higher tunability of the method according to the present invention, in an embodiment of the present invention, after separation of the carbamate C at step d), the carbamate C is further subjected to a transcarbamation reaction, thereby allowing to purposefully tune the properties of said carbamate e.g. to improve their behaviour at thermolytic conditions i.e. during a thermolysis reaction. To this extent, the carbamate C is reacted with a second alcohol G, and a carbamate H of the second alcohol G is formed. The second alcohol G can be either mono- or polyfunctional, and different from the first alcohol B provided at step b). The transcarbamation reaction 201 on the carbamate C provides for a carbamate H of the second alcohol G, and the first alcohol B. Subsequently, the carbamate H obtained is separated from the first alcohol B, and said carbamate is subjected to a thermolysis reaction 103, for the obtainment of an isocyanate E.

For example, the separated carbamate C can be subjected to a transcarbamation in the presence of an alcohol providing a resulting carbamate H with a higher or lower decomposition temperature, or with an alcohol providing a carbamate of which the thermolysis products are easier to separate.

In the context of the present invention, by means of the term "transcarbamation", reference is made to the chemical reaction where a carbamate having general formula R-NH-(C=O)-O-R', with R and R' different or the same, is modified at the -O-R' position by replacing said -O-R' group with a different alkoxy (-O-R") group. In other words, a transcarbamation involves the displacement of the -O-R' group of a carbamate by another -O-R" group. The transcarbamation can be accomplished for example by reacting the carbamate with an alcohol, such as a monofunctional alcohol R"-OH, or a diol, or triol etc... The carbamate subjected to the transcarbamation reaction can also be a dicarbamate, a tricarbamate etc., wherein one or more (C=O)-O-R' are modified at the -O-R' position by replacing said -O-R' group with a different alkoxy (-O-R") group.

For example, in accordance with an embodiment of the present invention, the method can comprise the steps of 1) subjecting to a glycolysis reaction a provided polyurethane, depolymerizing the polyurethane A and providing a first polyol comprising phase, and a second phase comprising a glycolysed carbamate, 2) separating 102 the glycolysed carbamate by means of phase separation from the polyol phase, 3) conducting a transcarbamation 201 on the separated glycolysed carbamate with a C₁-C₁₀ alcohol such as octanol, 4) separating 202 the obtained octanol modified carbamate therefrom, 5) performing a thermolysis reaction 103 on said octanol modified carbamate, thereby obtaining the alcohol F of the carbamate i.e. octanol, and an isocyanate, 6) separating 104 the isocyanate from the alcohol.

The method according to the present invention comprises the step e) of performing a thermolysis reaction 103 on the carbamate provided at step d), thereby obtaining an isocyanate E and an alcohol F.

The temperature at which the thermolysis reaction 103 is performed, has large implications on the yields, side-reactions, and downstream separability. The temperature and time of the thermolysis reaction are a balance between the following effects: 1) The thermal decomposition of the carbamate bonds into isocyanate groups and alcohols is favored by a higher temperature; e.g. 350 °C has a higher decomposition yield than 250 °C; 2) Side reactions such as the dimerization, trimerization and potentially self-condensation to carbodiimides are unwanted and occur in the presence of (trace) catalyst and at too long residence times, and are also favored by higher temperatures; 3) If a product is targeted that exists completely in the vapor phase, to then be separated downstream, a temperature above the boiling point of all decomposition products - isocyanates as well as alcohols - is preferred. For example a dicarbamate of MDI with ethylene glycol could be thermolysed for a short residence time at a temperature preferably exceeding 300 °C. For example a dicarbamate of TDI with ethylene glycol could be thermolysed at a temperature preferably exceeding 250 °C. The temperatures could be reduced by applying an entraining make-up gas; 4) If a liquid isocyanate resin is to remain in the reactor and potentially continuously extracted from the reactor, the thermolysis temperature should not exceed the boiling point of the lowest boiling isocyanate involved. This further depends on the presence of a makeup gas and the applied pressure. Further, in the case downstream separations are needed, the condensation/distillation temperature will depend on the combination of alcohols and isocyanates present in the carbamates, with a first separation unit operating at a temperature between the boiling points, followed by a full condenser to room temperature for the low-boiling product.

The alcohol F is the alcohol obtained from the thermolysis reaction 103 of the carbamate provided at step d), which alcohol originates from the -O-R' portion of the carbamate having general formula R-NH-(C=O)-O-R'. The alcohol F therefore corresponds to the first alcohol B, in case no transcarbamation reaction was performed, or the second alcohol G, or another alcohol obtained from said thermolysis e.g. in case side reactions occurred. Therefore, the alcohol F does not necessarily need to be either the first alcohol B or the second alcohol G, and can also be an alcohol having a different formula, e.g. due to side reaction on said -O-R' portion of the carbamate, taking place at high temperatures, such as rearrangements and elimination reactions.

In an embodiment, not according to the present invention, step e) comprises the steps of e2) providing a solution or suspension in a solvent S, of the carbamate, i.e. either carbamate C or the transcarbamation product, carbamate H provided at step d); e3) transferring to a thermolysis reactor, the solution or suspension provided at step e2); e4) performing the thermolysis reaction 103 in said thermolysis reactor.

In accordance with this embodiment, it has been found advantageous to firstly dissolve (or suspend), at least partially, the carbamate to be thermolysed at step e) in a solvent, and then provide said solution or suspension to where the thermolysis reaction 103 is provided to take place e.g. a reactor. In this way, the introduction of the carbamate e.g. in the reactor is facilitated, thereby allowing minimal loss of chemicals. During the thermolysis the solvent is provided to evaporate and not interfere with the thermolysis reaction 103. The solvent utilized is selected based on the properties of the carbamate to thermolyse and e.g. the solubility of the carbamate in said solvent. The selection of the solvent would therefore be evident to the skilled in the art.

In a further embodiment, not according to the present invention, at step e2), the solvent S is either the first or second alcohol G provided at step b) or d2). Therefore, the carbamate provided at step d) is provided at step e2) as a solution or suspension of the carbamate in a solvent S that is either the first alcohol B, provided to react with the polyurethane, and to alcoholyse said polyurethane, or the second alcohol G, provided to react with the carbamate, and perform a transcarbamation reaction with said carbamate. For example, a first alcohol B, such as ethylene glycol is provided and a glycolysis of a polyurethane is accomplished, thereby providing a product mixture comprising a polyol comprising phase and a carbamate comprising phase, wherein the carbamate comprising phase is an ethylene glycol mixture or solution, which is separated via split-phase process. The separated carbamate comprising phase is, in accordance with the present embodiment, transferred to a thermolysis reactor where thermolysis can take place, in the presence of said ethylene glycol.

In the context of the present invention, by means of the term "thermolysis", reference is made to the chemical reaction of thermal decomposition of one or more substrates, wherein one or more chemical bonds are broken by heating. In the context of the present invention, by means of the term "isocyanate", reference is made to a compound comprising at least one isocyanate functional group of formula R-N=C=O, wherein R can further comprise other -N=C=O groups. Therefore, by means of the term isocyanate reference is made to monoisocyanates, diisocyanates and triisocyanates etc.

In a further embodiment according to the present invention, at step e) the thermolysis reaction 103 is performed at a temperature from about 200 ºC to about 450 ºC, preferably from about 200 ºC to about 300 ºC.

In accordance with a particular embodiment of the present invention, the polyurethane A provided at step a) is a polyurethane made from MDI, and at step e) the thermolysis reaction is performed on a dicarbamate of MDI at a temperature from 230 ºC to about 280 ºC, preferably from 240 ºC to 270 ºC, more preferably around 250 ºC. It has been found that by means of the present embodiment it is possible to obtain the best results, as the present condition provide for the obtainment of the isocyanate thereby minimizing polymerization reactions.

In a further embodiment according to the present invention, at step e) the thermolysis reaction is performed at a pressure from about 1 bara (100 kPa) to about 10 mbara (1 kPa), preferably from about 1 bara (100 kPa) to about 100 mbara (10 kPa), more preferably from about 1 bara (100 kPa) to about 200 mbara (20 kPa), even more preferably from 1 bara (100 kPa) to about 0.9 bara (90 kPa); or under an inert gas such as nitrogen or argon. Nevertheless, in order to facilitate the separation of the isocyanate lower pressures can be used, such as 10mbara or lower.

Further, the method according to the present invention comprises the step f) of separating 104 the isocyanate E from the alcohol F, obtained at step d). It has to be understood that the alcohol F is the alcohol produced during the thermolysis of the carbamate. At step f), the isocyanate E can be separated from the alcohol F that is produced via the thermolysis reaction 103 by several means of several separation techniques, such as phase separation, filtration, distillation, chromatography, decantation. Preferably, the separation is accomplished by distilling away either the isocyanate E or the alcohol F, preferably the alcohol F. Preferably, said distillation is performed at reduced pressure.

In a preferred embodiment according to the present invention, at step f) the isocyanate E is separated from the alcohol F by distilling away the alcohol F at a temperature from about 100 ºC to about 300 ºC, preferably from about 200 ºC to about 250 ºC, more preferably from 210 ºC to about 230 ºC.

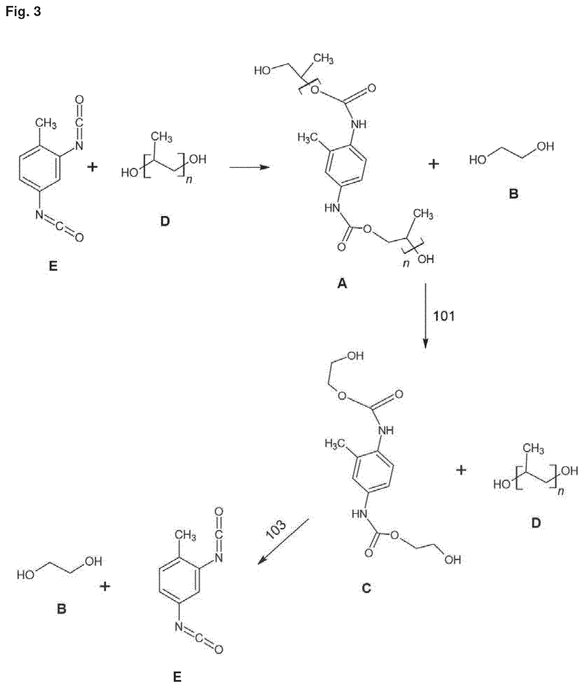

**Fig. 3** illustrates how the method of recycling according to the present invention can be put into practice. More specifically, **Fig. 3** illustrate how the method according to the present invention can be used to recycle a polyurethane foam comprising toluene diisocyanate (TDI) and polypropylene glycol (PPG) units. **Fig. 3** illustrates a polyurethane A, in this specific example a TDI-PPG polyurethane, reacted via alcoholysis 101 with a first alcohol B, in this specific example ethylene glycol, preferably in excess. The alcoholysis 101 provides for a carbamate C of said first alcohol B, in this specific example ethylene glycol toluene dicarbamate. By means of the alcoholysis reaction 101, a polyol D is also provided, in this specific example a PPG. After separation of the carbamate C, said carbamate is themolized, see 103, thereby forming an isocyanate E, in the present case a TDI and the first alcohol B, ethylene glycol. The present figure illustrates the full-cycle recycling provided by the method according to the present invention, wherein the components which can possibly be utilized to form the polyurethane A according the present example, meaning TDI and PPG, are obtained from the polyurethane, after alcoholysis 101 of said polyurethane, and thermolysis of the carbamate.

### EXPERIMENTAL PART

The present experimental part shows how the present invention can be put into practice. In particular, for the two-step process of the present invention, both steps, the alcoholysis of PU and the pyrolysis of carbamates, were initially investigated separately. Since the glycolysis of PU was already extensively discussed in the literature and even applied in pilot-scale plants, the pyrolysis reaction of carbamates was targeted first. In this way, the optimum conditions found for the pyrolysis reaction could be used as a starting point for the alcoholysis reaction at a later stage. The synthesis and pyrolysis of different dicarbamates was investigated.

### MATERIALS AND METHODS

### MATERIALS

### Used chemicals

4,4'-Methylenediphenyl Diisocyanate (99%, Pharmacia & Upjohn Company)
Isopropanol (99%, Fisher Scientific UK)
Silicone oil AP 100 (Aldrich)
tetraethylene glycol dimethyl ether (98%, Thermo Scientific)
Octanol (98%, Acros Organics - International)
Methanol (99.8%, Pharmacia & Upjohn Company)
Butanol (Sigma Chemical Company)
Ethylene glycol (99.8%, Sigma-Aldrich Brand Chemicals)
PU flexible foam

### METHODS

### DICARBAMATE GENERAL SYNTHESIS

MDI (mol, 5 g), the used alcohol (mol) and a magnetic stirring bar were brought into a round bottom flask (50 mL). The reaction mixture is then stirred at the boiling temperature of the alcohol for 2h. Thereafter the reaction was cooled to room temperature. The reaction product was washed with water and filtered via vacuum filtration. The remaining water was removed using a rotary evaporator and further dried in a vacuum oven for 12h. The dicarbamate product is then analyzed by liquid 1H-NMR (400 MHz) and SEC. Based on the methodology above, a series of dicarbamates were obtained, in particular, Carbamic acid, (methylenedi-1,4-phenylene)bis-, bis(2-hydroxyethyl) ester (EMd); Carbamic acid, (methylenedi-1,4-phenylene)bis-; dioctyl ester (OMd), Dimethyl 4,4'-methylenediphenylenedicarbamate (MeMd); Diisopropyl 4,4'-methylenediphenylenedicarbamate (IsoMd) and Dibutyl 4,4'-methylenediphenylenedicarbamate (BuMd).

### THERMOLYSIS OF DICARBAMATES

### General

The dicarbamate (0.5 g) and a magnetic stirring bar were brought into a round bottom flask (10 mL). When the heating mantle reached 250 °C the flask was placed in the heating mantle. The reaction mixture is then stirred at 250 °C for 5/15/30 minutes. The reaction was cooled using an ice bath. The product mixture is then analyzed by FTIR and SEC.

### Temperature variation experiments with EMd

1.0 g of EMd was ground into a powder. Then, this powder was added to a three-necked 100 mL Schlenk flask through a funnel. In addition, a magnetic stirrer was also added and set to 200 rpm. Next, the flask was connected to the setup, comprising an adapted Liebig cooler connected to a cooling both and a downstream Erlenmeyer to collect condensed liquid. The setup was brought under argon atmosphere. During this experiment four different temperatures were investigated namely: 220°C, 250°C, 285°C and 300°C. When temperature was reached the reactions were carried out for 20 minutes. Afterwards, the contents of the flask, condenser, and if needed (i.e. if condensate was present) the Erlenmeyer flask were analyzed with FTIR.

### SOLVENT ASSISTED THERMOLYSIS OF DICARBAMATES

Diisopropyl 4,4'-methylenediphenylenedicarbamate (0.5 g) and a magnetic stirring bar were brought into a 2-neck round bottom flask (10 mL) under Ar-flow. When the oil bath reached 236 °C the flask was placed in the oil. The reaction mixture is then stirred at 236 °C- 240 °C for 15 minutes. After the reaction was finished the flask was removed from the set-up and placed in an ice bath. The product mixture is then analyzed by FTIR and SEC.

### ALCOHOLYSIS OF FLEXIBLE PU FOAM

### Alcoholysis using octanol

To a two-neck flask, 10.000 g of octanol was added and placed in an argon atmosphere with a condenser for 15 min. Thereafter, the flask was heated to 180 °C and 5.000 g of flexible foam was added through the solid loader slowly. When a homogenous mixture formed, the flask was sealed with an argon atmosphere and heated up to 200 °C for 2 h. Lastly, the depolymerized product was collected and separated the octanol carbamate through Flash chromatography tested on GPC, and NMR.

### Alcoholysis using isopropanol

An autoclave with a pressure capacity of up to 200 bar was used in this experiment. First of all 15.000 g of isopropanol and 3.000 g of flexible foam were charged to the autoclave reactor and sealed and fastened with a hex key. The autoclave was purged with argon for 15 min. The pressure of the autoclave was increased to 30 bar with argon. At this pressure, the reaction mixture was heated to 200 °C for 2 h. Lastly, the reaction mixture cooled to room temperature and pressure was released. The depolymerized product was separated on flash chromatography to get isopropanol carbamate.

### DICARBAMATE ISOLATION

### OMd isolation

Crude sample (3 g) was dissolved in 50 mL acetone. This was added to 15 g silica and dried with a rotary evaporator. The contents were placed in a solid load cartridge. The column used is a generic silica 24g, solvent A corresponds to n-heptane and solvent B to ethyl acetate.

| Gradient Table | | | |
|---|---|---|---|
| | Min | Solvents | % 2^{nd} |
| 1 | 2.0 | AB | 0 |
| 2 | 10.0 | AB | 25 |
| 3 | 5.0 | AB | 50 |
| 4 | 23.0 | AB | 100 |

### IsoMd isolation

Crude sample (6 g) was dissolved in 50 mL acetone. This was added to 15 g silica and dried with a rotary evaporator. The contents were placed in a solid load cartridge. The column used is a generic silica 24g, solvent A corresponds to n-heptane and solvent B to ethyl acetate.

| Gradient Table | | | |
|---|---|---|---|
| | Min | Solvents | % 2^{nd} |
| 1 | 2.0 | AB | 0 |
| 2 | 10.0 | AB | 25 |
| 3 | 10.0 | AB | 50 |
| 4 | 13.0 | AB | 100 |

### EXAMPLE 1 - PYROLYSIS OF DICARBAMATES

### - SYNTHESIS OF MODEL DICARBAMATES

Since MDI is one of the most frequently used isocyanates in PU, the initial research was focused on MDI based dicarbamates. For the synthesis of these dicarbamates, different alcohols were selected based on their reactivity and selectivity in the thermolysis reaction. These alcohols can react directly with MDI to form the dicarbamate. The alcohols used for synthesis will consist of mono-alcohols with varying chain length and branching. The limitation to mono-alcohols is made to exclude polymerization to form PU. Other isocyanates like TDI, HDI and polymeric MDI will also be used as isocyanates for the formation of these model compounds. The selected isocyanates are MDI, TDI and HDI, whereas the selected alcohols are isopropanol, butanol, octanol and methanol. The structure and purity of the obtained components was checked by proton nuclear magnetic resonance (¹H NMR) and size exclusion chromatography (SEC).

### - THERMOLYSIS SYNTHESIZED DICARBAMATES

Using a simple laboratory setup, the different synthesized model components are subjected to the thermolysis reaction. The setup consists of jacket-heated thermolysis chamber. The exit of the chamber is connected through a water-cooled condenser to a cooled recipient under vacuum. The thermolysis is performed at different temperatures, different residence times and under vacuum (below 10 mbar).

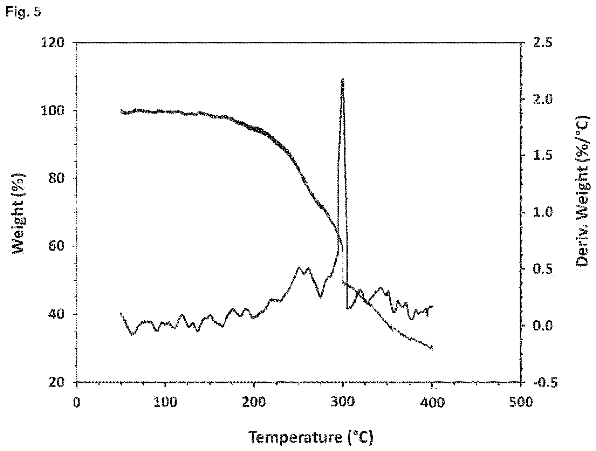

The thermolysis is initially tested at 150, 200 and 250 °C, as the dissociation temperature of the urethane bond for alcohols and isocyanates is approximately 250 °C for aliphatic alcohols and isocyanates and 180 °C for aliphatic alcohols and aromatic isocyanates 8,9. These values were also supported by the TGA analysis of Bis(2-hydroxyethyl)(methylenebis(4,1-phenylene))dicarbamate (EMd), one of the model dicarbamates that was synthesized. **Fig. 5** illustrates results of TGA analysis (thermal gravimetric analysis) for EMd. In the figure, the TGA curve is plotted starting from the top left (100% weight), whereas the other curve, represents the DTG curve (first derivative of the TGA curve).
From **Fig. 5****,** it can be seen that the mass loss starts around 200°C, reaching a maximum rate at 300°C. This is confirmed by taking the first derivative of the TGA curve (DTG curve).

The thermolysis reaction time is varied to find the optimum reaction time at which maximum carbamate dissociation occurs and as little isocyanate as possible reacts to produce undesirable by-products. Based on already conducted experiments, residence times of 1, 5, 15 and 30 minutes are tested initially. By working under vacuum, the residence time of the released gaseous alcohols is reduced and the back-reaction thus also prevented.

By performing the thermolysis reaction several times, a set of standard reaction parameters was set. It was noted that at 250 °C, 15 minutes and at a pressure of less than 10 mbar, the best results were obtained. In testing the various carbamates, these reaction conditions were therefore used as a starting point.

### - STRATEGY

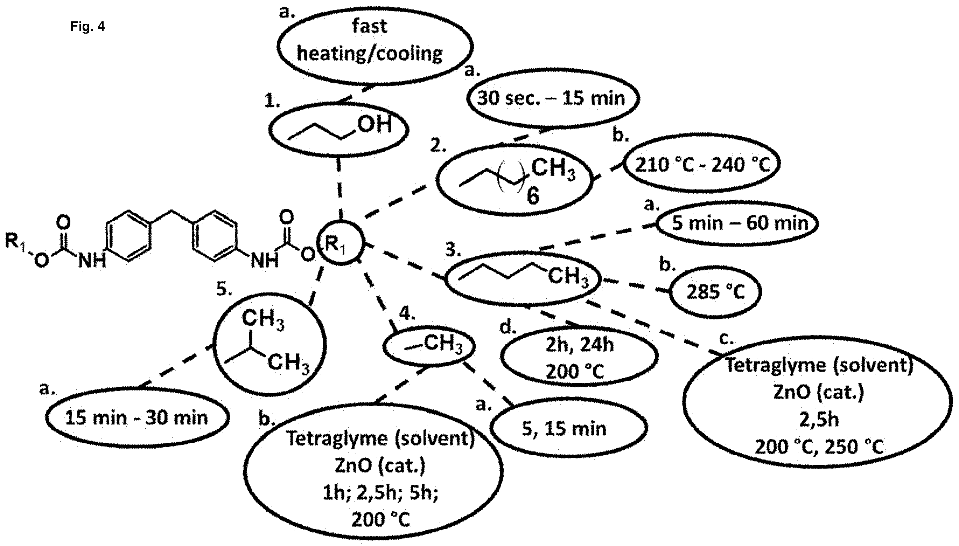

To establish the standard reaction conditions, several thermolysis reactions were tested, as shown in **Fig. 4****.**

Initially, this research focused on ethylene glycol as the alcohol used in the synthesis of the dicarbamate (reaction 1). Ethylene glycol was initially chosen for the carbamate synthesis due to the fact that this molecule is already used on a small scale in industry for the glycolysis of PU. In the glycolysis reaction, a two-phase mixture is formed with an apolar polyol phase and a polar phase containing the ethylene glycol based dicarbamate. The carbamate present in the polar phase could then be isolated and used directly in the thermolysis reaction to regenerate isocyanates. This avoids the need for an intermediate step in the process where the carbamate obtained from the glycolysis reaction would have to be converted to another carbamate for the thermolysis reaction through a transcarbamation reaction. One disadvantage of using ethylene glycol is the bifunctionality of the molecule. This led to a polymerization reaction during thermolysis to form polyurethane oligomers and prevented the recovery of the isocyanate. This polymerization reaction can be avoided by using monofunctional alcohols.

A first monofunctional alcohol tested was octanol (reaction 2). This alcohol was selected because of its similar boiling point to ethylene glycol (around 200 °C) which makes it suitable for the alcoholysis reaction and the carbamate obtained, as with the use of ethylene glycol, can be used directly for the thermolysis reaction. An additional advantage in using octanol as an alcohol is the steric hindrance of the long aliphatic chain. This will hinder the back-reaction of the isocyanate group and alcohol group to the carbamate. For the thermolysis of the octanol-based carbamate, different reaction times (reaction 2a) and temperatures (reaction 2b) were also tested to find the ideal reaction conditions. Although the use of monoalcohols allowed the exclusion of the polymerization reaction to polyurethane oligomers, the alcohol does remain present in the reaction mixture during the reaction. This allows the highly reactive isocyanates formed to react back with the alcohol and the carbamate is formed again. One solution to this problem is to use alcohols with a lower boiling point, that can be evaporated during the thermolysis reaction, such as butanol (reaction 3).

Butanol was selected since this alcohol has a lower boiling point of 117.7°C, which is lower than that of octanol but still allows a reaction temperature of 100°C for the alcoholysis reaction. The reaction time was also varied for the thermolysis reaction of butanol (reaction 3a, 3b, 3d). The influence of a solvent and catalyst on the reaction was also tested. Although butanol has a lower boiling point than octanol, the alcohol was not sufficiently removed from the reaction mixture. Due to this observation, monoalcohols with an even lower boiling point such as methanol (reaction 4) were examined.

The thermolysis of methanol was tested with different reaction times (reaction 4a) and again the effect of a catalyst and solvent was evaluated (reaction 4b). Although methanol was efficiently removed from the reaction mixture by evaporation, the alcohol was not suitable for an effective dissociation of the carbamate. Thus, finally, isopropanol was tested as an alcohol (reaction 5). Isopropanol was selected due to the low boiling point of the alcohol (82.5 °C) and the fact that it is a secondary alcohol. Secondary alcohols, according to the literature, would cause the carbamate to dissociate more efficiently.

During a first characterization step, the obtained thermolysis product mixture is analyzed by combining Fourier-transform infrared (FT-IR) spectroscopy and size exclusion chromatography (SEC), before being separated into the different product fractions. The presence of the NCO-group of the isocyanate will be confirmed by Fourier-transform infrared (FT-IR) spectroscopy due to the fact that the NCO group of isocyanates exhibits strong absorption between 2250 cm⁻¹ and 2270 cm⁻¹, the appearance of a peak in this region thus confirms the presence of this group. Coupling this information with the SEC analysis where MDI has a characteristic retention time of 17 minutes, we can confirm the presence of MDI in the reaction mixture. This initial characterization aims to confirm the presence of MDI in the reaction mixture in order to obtain a first set of standard reaction parameters for pyrolysis.

### - PYROLYSIS OF ETHYLENE GLYCOL-BASED DICARBAMATES AND INVESTIGATION OF EFFECT OF DIFFERENT TEMPERATURES

A thermolysis reactions (1a), (see top of Fig. 4) was performed with Carbamic acid, (methylenedi-1,4-phenylene)bis-, bis(2-hydroxyethyl) ester (EMd). An alternative name for EMd is Bis(2-hydroxyethyl)(methylenebis(4,1-phenylene))dicarbamate.
The thermolysis was performed at 250°C for 15 minutes under vacuum. The FTIR spectrum of the obtained reaction mixture shows at the region between 2400 cm⁻¹ and 2200 cm⁻¹ no peaks present. This indicates that there are no isocyanate groups present in the reaction mixture.

The SEC chromatogram shows large peaks at low retention times, which indicates the formation of oligomers and polymers. This can be explained by the bifunctionality of ethylene glycol which makes it possible for polymerization to occur and so to form polyurethane oligomers. The peak at retention time of 15.1 is the starting compounds EMd. The peak present at 18.5 minutes is presumed to be ethylene glycol and the peak at 15.9 is still unknown. The wide peak around 17 minutes is not MDI but could be due to some impurities in the SEC solvent.

The thermal decomposition reaction of EMd is schematically illustrated according to the reaction scheme here below:

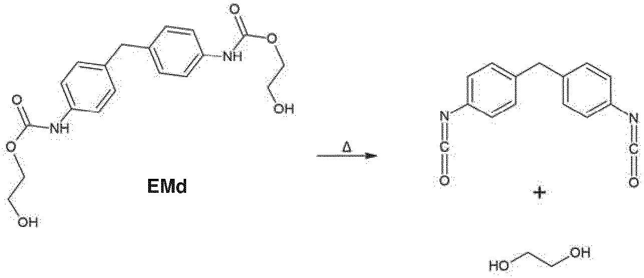

To determine the optimal reaction temperature, a series of thermolysis reactions were performed at different reaction temperatures.

### 220°C

The first of four experiments was carried out at 220°C. The FTIR spectrum of the residue in the flask did not show the characteristic peak of isocyanate, likely because of side reactions such as polymerization during the gradual evaporation of ethylene glycol, altering the stoichiometry.

### 250°C

Next, the experiment was carried out at a temperature of 250°C. During the reaction, droplets were formed that slid down the walls of the flask onto the solid product. A sample of the solid/liquid product was taken and analyzed. The FTIR result of the collected sample shows a peak at 2250 cm⁻¹, belonging to the isocyanate (MDI) (data not shown). Further analysis of the spectrum shows that carbamate bonds seem to be present, probably the solids.

### 285°C

The thermal decomposition at 285°C also did not deliver free isocyanates nor a carbodiimide (product of self-addition of isocyanate at high temperature). A small amount of ethylene glycol was found on the condenser, proving that thermal decomposition occurred, but the residue in the flask polymerized (not soluble in common solvents).

### 300°C

Similarly to the thermal decomposition at 285°C this experiment did not deliver free isocyanates nor a carbodiimide. At 300 °C even more ethylene glycol was found on the condenser but again the residue in the flask polymerized.

Table 1 gives an overview of the mass loss during the thermolysis experiments at various temperatures, by weighing the sample in the flask before and after the thermolysis reaction. It is clear that the mass loss increases with temperature until a level of approx. 50 % by mass. Note that this 50 % by mass is more than solely the mass of the ethylene glycol, which should be approximately 33 % of the overall mass. This means that likely some of the formed isocyanates are also evaporated.

**Table 1**

| Reaction temperature (°C° | Mass sample (g) | Mass after reaction (g) | Mass loss (%) |
|---|---|---|---|
| 220 | 1.2753 | 0.8431 | 33.89 |
| 250 | 0.9135 | 0.4860 | 46.79 |
| 285 | 0.9296 | 0.4655 | 49.92 |
| 300 | 1.030 | 0.5209 | 49.43 |

### - PYROLYSIS OF OCTANOL-BASED DICARBAMATES

Subsequently, a pyrolysis was performed with Carbamic acid, (methylenedi-1,4-phenylene)bis-, dioctyl ester (OMd), synthesized by a reaction of MDI with octanol. The reaction equation of said pyrolysis is schematically represented herein below.

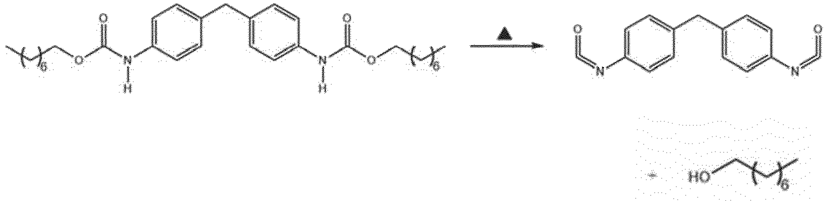

The thermolysis reaction was performed at 250°C under vacuum for 0, 1, 5, 10 and 15 minutes, taking 1 minute into account for the heating of the mixture. After the reaction the mixture is immediately cooled using an ice bath and solid fraction left in the flask and the condense trapped in the connecting piece, are analyzed via FTIR and SEC.

On the FTIR spectrum (not shown) of the solid fraction, a clear peak can be observed at 2250 cm⁻¹ which confirms the presence of the NCO group in the mixture. However, this peak could also originate from a molecule where only "one arm" of the carbamate is dissociated to form NCO and the other arm still contains a urethane compound. The sharp peak at wave number 3300 corresponds to N-H stretching of the carbamate and the 3 peaks at 3000 cm⁻¹ to 2800 cm⁻¹ correspond to C-H stretching of the carbamate.

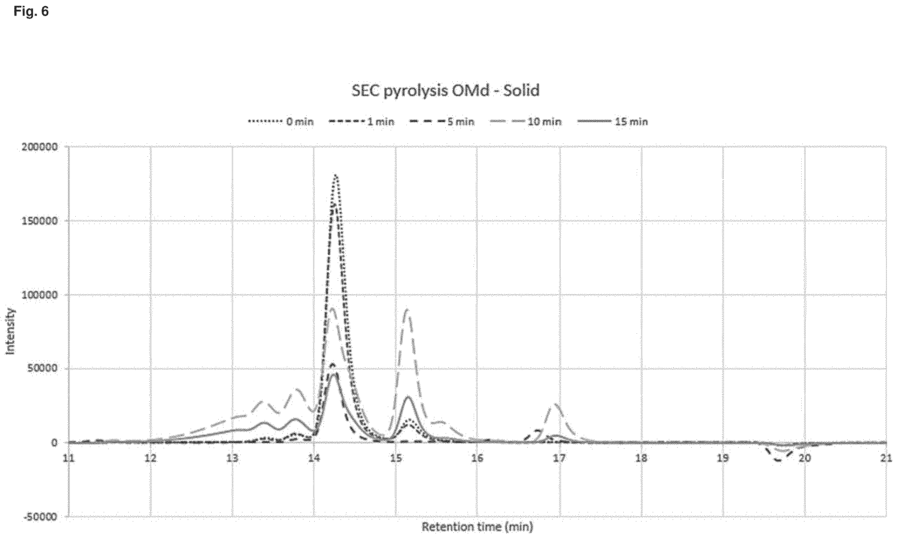

The presence of MDI can be confirmed by SEC, where the peak at 17 minutes corresponds to a molecular mass equal to the molecular mass of MDI. On the chromatogram **(****Fig. 6****)** other peaks are present at 14.2 and 15.1 minutes these peaks correlate to the starting compound OMd and the intermediate with isocyanate group on one side and octyl carbamate group on the other side. The formation of this intermediate requires less energy than the formation of MDI itself. This is a stable intermediate because the dissociation of the remaining carbamate group is complicated by electronic effects.

The peaks with retention times less than 14 minutes are assumed to be oligomers produced as by-products during pyrolysis. From the spectra and the chromatogram, it can be concluded that only after 5 minutes a complete dissociation of the carbamate to MDI has taken place The structure determination of the different components of the reaction mixture as well as the quantification of the produced MDI will be accomplished through additional analysis techniques.

When analyzing the condensate from the various pyrolysis reactions, it was noticed from both the SEC chromatogram and the FTIR spectrum that no MDI is present in these fractions. The condensate from the 0 and 1 minute pyrolysis reactions did not contain enough sample for FTIR analysis. The peak in the SEC chromatogram at 16.7 minutes corresponds to the octanol that is present in the vapor fraction.

As the SEC chromatogram of the solid fraction indicates, a whole number of oligomers are formed during the thermolysis reaction. To counteract this oligomerization, thermolysis experiments with OMd were performed at lower temperature, 210°C to 240°C for 1 and 5 minutes. However, during the analysis it appeared that only a small amount of carbamate was dissociated, that the oligomerization was not prevented and that on top of that no MDI is produced during these reactions. One explanation may be that at these temperatures the released octanol does not evaporate and immediately reacts back with MDI to form the carbamate. The free NCO group may also react with another NCO group to form an oligomer. Although the formation of MDI by thermolysis is confirmed, a large number of side products are clearly present in the reaction mixture. Hence, to increase the MDI selectivity of the reaction, other alcohols must be considered.

### - PYROLYSIS OF BUTANOL-BASED DICARBAMATES

A second carbamate tested for the thermolysis reaction is Dibutyl 4,4'-methylenediphenylenedicarbamate (BuMd). Compared to octanol, butanol has a lower boiling point (117 °C), which facilitates the evaporation of the alcohol during pyrolysis and promotes the necessary separation between the alcohol and MDI. The reaction was carried out at 250°C for 5, 15 and 30 minutes under vacuum. Analysis of the reaction mixture was performed via FTIR and SEC.

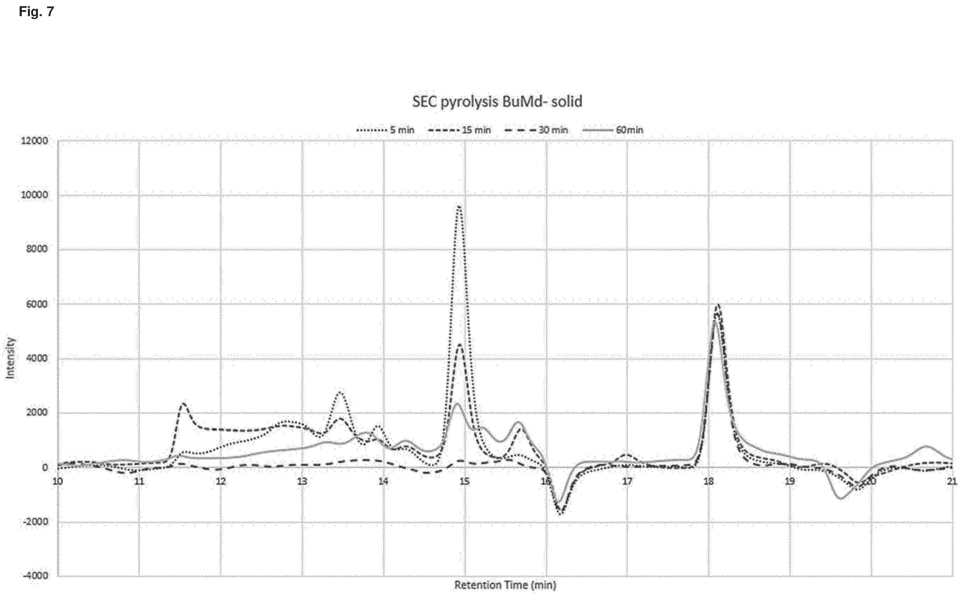

From the SEC chromatogram **(****Fig. 7****)** we can see that only a small amount of the intermediate with isocyanate group on one side and butyl carbamate group on the other side is formed (peak at 15.7 minutes) after 5 and 15 minutes of thermolysis. Only when reacting for 15 minutes a very limited amount of MDI is found in the mixture. During the reactions of 5 and 15 minutes, a considerable amount of starting material is still present in the reaction mixture (peak at 15 minutes) and a large variety of oligomers are formed. An explanation for these results can also be found in the chromatogram by the peak that appears at a retention time of 18 minutes. This retention time correlates with the presence of butanol in the reaction mixture. Due to the presence of butanol, the released NCO group will quickly react back with the alcohol and the oligomers are formed by the reaction of two NCO groups. The poor separation of butanol from the reaction mixture could be explained by poor performance of the vacuum pump in the setup. If the pressure in the flask is not reduced sufficiently, the evaporation of butanol will be less efficient and more butanol will remain in the flask. In the 30 minute reaction, apart from the peak of butanol, there are hardly any peaks observable in the chromatogram. This is because at this long reaction time, polymers were formed in the flask that were not soluble in THF. The negative peaks in the chromatogram are due to impurities present in the THF used.

The infrared spectrum of the solid fraction (not shown) confirmed the assumptions made based on the SEC chromatogram. The peak at 3300 cm⁻¹ and the peaks at 3000 cm⁻¹ to 2800 cm⁻¹ can be attributed to the carbamate. The characteristic peak of the NCO group is also present and can be linked to the small amount of MDI (in the case of the 15 minute thermolysis reaction) and to the one arm carbamate. At a wavelength of 2100 cm⁻¹, a new peak appears that originates from the symmetrical and asymmetrical stretching of carbodiimide group (R-N=C=N-R), formed in the polymerization process.

The SEC chromatogram of the collected condensate fraction (not shown) does show a clear MDI peak at reaction times of 15 and 30 minutes. Also the peaks of the starting product and the one-arm carbamate are clearly present. This shows that all three components can evaporate at the thermolysis conditions used. The oligomers present at a reaction time of 30 minutes are assumed not to have evaporated but formed in the connecting piece. At a reaction time of 5 minutes, two small peaks can be observed from the starting material and the one-arm carbamate in addition to butanol. The negative peaks at retention times 16.1 and 19.8 are again from impurities in the THF used.

To suppress the polymerization reaction during thermolysis, a thermolysis reaction was performed at lower temperature because the polymerization reaction could be promoted at higher temperatures. To compensate for this lower temperature, longer reaction times were applied, 2 hours and 24 hours. However, when analyzed the SEC and FTIR results showed that no MDI was formed and that mainly BuMd was present in the reaction mixture.

### - PYROLYSIS OF METHANOL-BASED DICARBAMATES

Since thermolysis with butanol-based dicarbamates was not efficient for the production of MDI during the thermolysis reaction, another alcohol was again called upon. This time a methanol-based dicarbamate was synthesized, Dimethyl 4,4'-methylenediphenylenedicarbamate (MeMd). Methanol was selected as the alcohol because due to its low boiling point it will evaporate very easily once it is released during the reaction. This will prevent the back reaction to the carbamate.

The thermolysis of MeMd was carried out like previous experiments with the other cabamates at 250°C under pressure for 5, 15 and 30 minutes. However, compared to the experiments with the other carbamates, a clear MDI peak was not observed in the SEC chromatogram (not shown) for MeMd at any of these reaction times. This chromatogram shows that only very limited amount of MeMd was reacted. However, the peak of MeMd at a retention time of 15.8 minutes shows a slight broadening along the right side. This may be a result of the limited formation of the one-arm carbamate which, due to the limited molecular weight of methanol, possesses approximately the same retention time as MeMd. There are small peaks around retention times of 14 and 15 minutes indicating that oligomers have formed. The fact that oligomers have formed even when no MDI is present in the mixture can be explained by the formation of the one-arm intermediates that can react with each other to form oligomers. The FTIR spectrum confirmed the assumptions made based on the SEC chromatogram.

### - PYROLYSIS OF ISOPROPANOL-BASED DICARBAMATES

Since the pyrolysis with the methanol-based carbamate were not successful, another low boiling alcohol was used for the carbamate synthesis, isopropanol. An additional advantage of isopropanol is the fact that it is a secondary alcohol, and carbamates derived from secondary alcohols can dissociate at lower temperatures than primary alcohols due to the steric hindrance around the hydroxyl functionality.

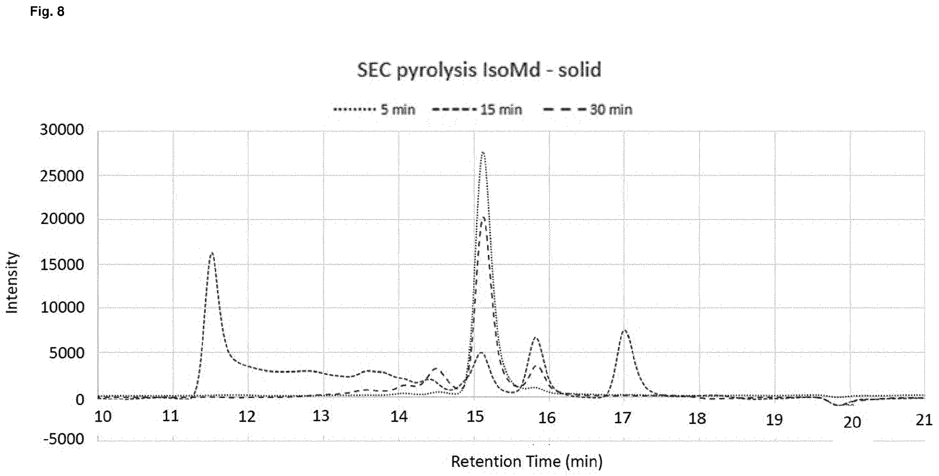

The thermolysis of Diisopropyl 4,4'-methylenediphenylenedicarbamate (IsoMd) was carried out like previous experiments with the other cabamates at 250°C under pressure for 5, 15 and 30 minutes. The SEC chromatogram **(****Fig. 8****)** shows that in the pyrolysis reaction after 5 minutes mainly IsoMd is still present. In the 15 minute reaction, a very clear peak at 17 minutes can be seen corresponding to MDI. Also a very broad peak in the polymer region can be observed indicating different oligomers of different chain length. The sharp peak at 11.5 minutes arises due to the limitation in observable chain lengths of the column used. As a result, molecules that have a chain length that correlates with a retention time greater than 11.5 minutes all come out of the column at the same retention time. At a reaction time of 30 minutes, polymers were formed that no longer dissolved in THF and thus were not observable in the chromatogram. The reason behind the large amount of IsoMd present in the reaction mixture may be due to a blockage of the three-way valve used in the setup. Throughout the different reactions a small amount of carbamate is volatilized and accumulates in the tubes of the reaction set-up. This causes a reduced performance of the vacuum pump resulting in the presence of released alcohol in the flask.

The infrared spectrum (not shown) confirmed the assumptions made based on the SEC chromatogram.

By testing the various dicarbamates, it became clear that the isolation of the released alcohol during the thermolysis reaction is essential for the synthesis of MDI. This isolation depends on the alcohol used for carbamate synthesis, the temperature and pressure in the flask. It is important to keep the reaction going long enough for MDI to form but not too long so that it has time to polymerize. Based on the analysis method used, thermolysis of the isopropanol-based dicarbamate at 250 °C for 15 minutes and with a pressure below 10 mbar results in the largest MDI production. In a next phase of the study, this assumption will be investigated by means of a quantitative high-performance liquid chromatography (HPLC) method and confirmation of the molecular structures present in the reaction mixture via 1HNMR.

### - QUANTITATIVE ANALYSIS OF THERMOLYSIS PRODUCTS

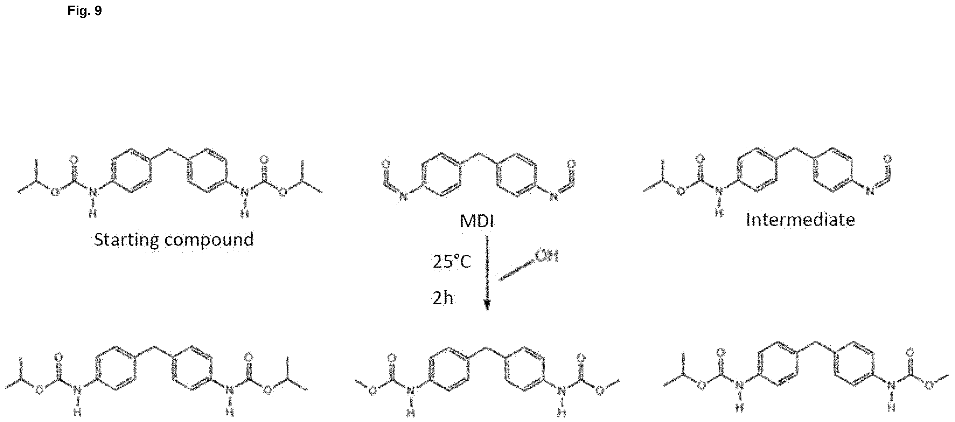

To determine the yield of the thermolysis reaction, a quantitative analysis method was established. The quantitation analysis uses an external standard HPLC method. Where by means of a calibration curve obtained in advance, it can be determined how much mass of a particular component is present in the mixture. However, before this HPLC method can be performed, a derivatization of the product mixture must be performed. This derivatization is necessary because the obtained isocyanate groups in the mixture are highly reactive and may react with the eluent used, the column, or even the moisture present in the air with which it may come into contact during sample preparation. This derivatization is obtained by again adding an alcohol to the reaction mixture. This blocks the reactive isocyanate group and a carbamate is obtained again. The alcohol used for the blocking reaction is methanol. Methanol was chosen because it is a good solvent for the reaction mixture obtained and because it is also used as an eluent during HPLC analysis. A schematic overview of the blocking reaction of theisocyanate functionalities present in the reaction mixture is presented in **Fig. 9****.**

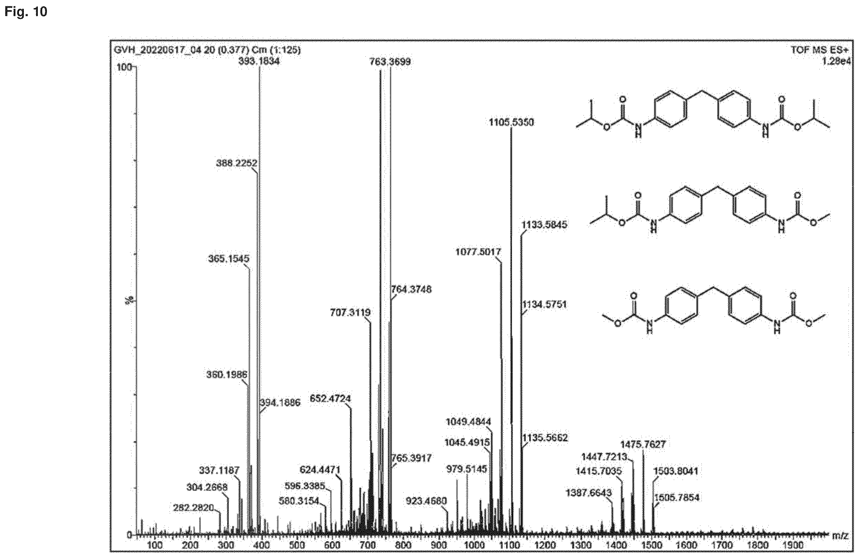

HPLC analysis was performed on the reaction products obtained after pyrolysis of IsoMd at 250 °C for 15 min under vacuum. A reverse phase column and an eluent composition of methanol/water (70/30) were used for the HPLC analysis. The first peak in the chromatogram (not shown) is from the methanol-based carbamate and the corresponding mass percentage should therefore also be seen as the mass percentage of MDI originally present in the reaction mixture. The second and third peaks are from the intermediate and starting component. The remaining peaks in the chromatogram are assumed to be the oligomers formed during the reaction. The exact structure of these oligomers remains to be investigated.
The mass spectrum of the reaction mixture was also analyzed on a Time-of-flight mass spectrometer (TOF-MS) after the HPLC analysis. Through the masses obtained in the spectrum (Fig. 10), it can be concluded that the molecules described above are effectively present in the reaction mixture. Thus, the peak at 393.1834 corresponds to the sum of the mass of IsoMd (370.19) and a sodium ion (22.99), the peak at 365.1545 corresponds to the mass of the intermediate (342.16) and a sodium ion, and the peak at 337.1187 correlates with the mass of MeMd and a sodium ion. These sodium ions present are probably from the glassware used.

### - SOLVENT ASSISTED THERMOLYSIS

Previous thermolysis reactions were always performed in the absence of a solvent. However, adding a solvent to the thermolysis reaction has some advantages. For example, the solvent present dilutes the concentration of the reactive isocyanates and alcohols so that the back reaction and side reactions can be suppressed. Solvents also enhance heat transport. However, a good solvent for thermolysis must have a boiling point higher than the reaction temperature, must not contain any reactive groups, and must be a good solvent for the carbamates. However, one drawback of using solvents is the additional separation of the solvent and reaction products after the reaction is completed.

For this study, two different solvents were tested for the thermolysis reaction namely silicone oil -[Si(CH₃)₂-O]ₙ- and tetraethylene glycol dimethyl ether. Silicone oil was selected based on its very high boiling point (about 300 °C) and tetraethylene glycol dimethyl ether contains ether functions in its structure which according to the literature would promote the thermolysis of carbamates.

Because both solvents would evaporate when thermolysis was performed at 250 °C under vacuum, it was chosen to work under an argon flow. Thus, the thermolysis can still be performed at 250 °C but the solvent will not evaporate completely.

The infrared spectrum (not shown) of both reactions shows a clear peak at 2300 cm⁻¹ which corresponds to the signal of isocyanate functionality. As with previous reactions, this signal can originate from either MDI or the intermediate.

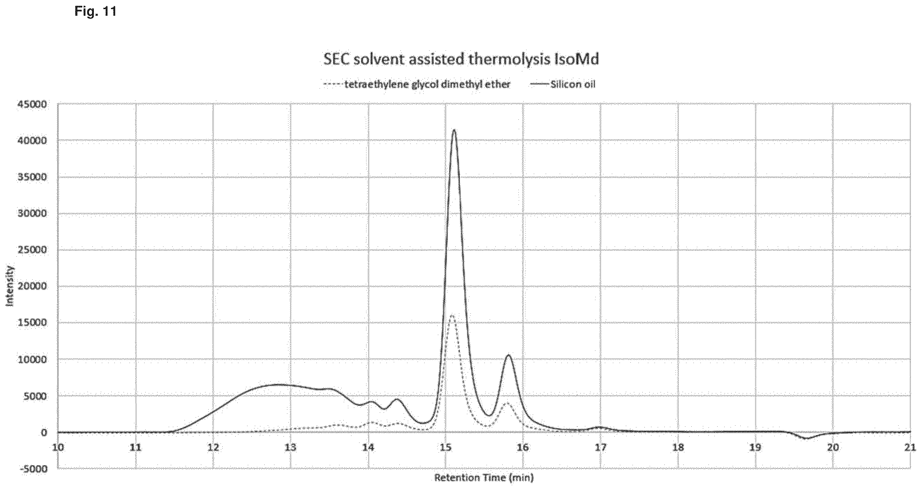

By analyzing the SEC chromatogram **(****Fig. 11****),** it can clearly be concluded that the starting material IsoMd, with a retention time of 15.1, is still largely present in the reaction mixture. A second strong peak at a retention time of 15.8, is that originating from the intermediate. At even longer retention times, a small peak can be observed at 17 minutes, this is the peak corresponding to MDI. It can also be seen from the chromatogram that oligomers were formed by the broad peaks at retention times 12 to 14.5.

Based on the FTIR spectrum and SEC chromatogram, we can conclude that MDI is effectively formed during the solvent assisted thermolysis reaction. However, this presence appears to be very limited at first glance. A possible explanation for this can be found in the reaction temperature during thermolysis. This was not 250 °C but only 240 °C due to a problem with the used oil bath. This problem can be solved by using another heat source. If this adjustment to the reaction turns out to be insufficient, other parameters such as reaction time, atmosphere and any catalyst can also be adjusted for optimization of the reaction.

### EXAMPLE 2 - THE ALCOHOLYSIS-THERMOLYSIS PROCESS

Now that the optimal reaction conditions and structure of the dicarbamate have been found, this information can be applied in the formulation of the entire two-step recycling process from PU to isocyanates that consists of 3 steps: the alcoholysis reaction, isolation of the formed dicarbamates, thermolysis of the dicarbamates.

### - ALCOHOLYSIS OF FLEXIBLE PU FOAMS

The alcoholysis was tested on flexible PU foam. Two different alcohols, octanol and isopropanol, were used for the alcoholysis. Both reactions were carried out at 200 °C for 2 hours. For octanol, the reaction was performed in an inert atmosphere by using an argon flow. Since the boiling point of isopropanol is 82 °C, this alcoholysis was performed under pressure.
The composition of the reaction mixtures was analyzed via SEC (data not shown). Both alcoholysis have a strong broad peak at 11 minutes, this is the peak coming from the polyols present in the reaction mixture. The peak at the alcoholysis reaction with isopropanol of 15.1 corresponds to the isopropanol carbamate, the other peaks in the spectrum need further analysis before they can be identified. For the alcoholysis reaction with butanol, the peak at 14.2 here corresponds to the octanol carbamate and as with the isopropanol reaction, the remaining peaks in the spectrum need further analysis for identification.

### - ISOLATION OF DICARBAMATES

After confirming the formation of both carbamates, a separation method by flash chromatography was established. This was based on the fact that the carbamates were most likely the most non-polar compounds in the reaction mixture, a hypothesis that was first confirmed via TLC.

The first fraction obtained from the flash chromatography separation of the reaction mixture from the isopropanol alcoholysis of PU was collected and the solvent was evaporated. The obtained white solid in this first fraction was analyzed via SEC and HNMR which confirmed the presence of pure IsoMd.

The same process was carried out for the products of octanol alcoholysis and from this chromatogram as well, the first fraction was isolated and analyzed via SEC.

### - THERMOLYSIS OF DICARBAMATES

After the isolation of IsoMd and OMd, they are immediately subjected to the thermolysis reaction. This reaction is carried out for both dicarbamates at 250°C for 15 minutes under vacuum. As before, the analysis of the thermolysis products was done via FTIR and SEC.
FTIR and SEC chromatograms (not shown) of the pyrolysis products of both carbamates were obtained. Based on these, for the thermolysis of IsoMd, it can be clearly established that MDI is formed by the presence of the isocyanate peak in the FTIR spectrum and the peak with a retention time of 17 minutes in the chromatogram. In the thermolysis of OMd, there is also a very strong peak of isocyanate function present in the FTIR spectrum but only a small peak of MDI in the chromatogram. This may be due to the limited amount of OMd at the beginning of the thermolysis reaction. A second thermolysis with 0.5 g OMd would have to be carried out to give a definite answer as to the efficiency of the reaction.

### Legend

A: polyurethane
B: first alcohol
C: carbamate of said first alcohol B
D: polyol
E: isocyanate
F: alcohol, deriving form carbamate thermolysis, that can be either the first alcohol or the second alcohol
G: second alcohol
H: carbamate of the second alcohol

## Claims

1. A method (100) of recycling of a polyurethane, comprising the steps of:
a) providing a polyurethane A;
b) providing a first alcohol B, either polyfunctional or monofunctional selected from the list comprising: propanol, pentanol, isopropyl alcohol, butanol, hexanol, nonanol, octanol;
c) performing an alcoholysis reaction (101) of the polyurethane A provided at step a) with the first alcohol B provided at step b), thereby obtaining a carbamate C, and a polyol D;
d) separating (102) the carbamate C from the polyol D, obtained at step c), thereby providing the carbamate C;
e) performing a thermolysis reaction (103) on the carbamate provided at step d), thereby obtaining an isocyanate E and an alcohol F, which alcohol F is a product of said thermolysis reaction on the carbamate provided at step d); and
f) separating (104) the isocyanate E from the alcohol F.

2. The method (100) according to claim 1, wherein at step b) the first alcohol B is ethylene glycol.

3. The method (100) according to any one of the previous claims, wherein step d) further comprises the steps:
d2) providing a second alcohol G, either mono- or polyfunctional, different from the first alcohol B provided at step b);
d3) conducting a transcarbamation reaction (201) on the carbamate C provided at step d), thereby obtaining a carbamate H of the second alcohol G provided at step d2), and the first alcohol B;
d4) separating (202) the carbamate H obtained at step d3) from the first alcohol B.

4. The method (100) according to any one of the previous claims, wherein at step e) the thermolysis reaction (103) is performed at a temperature from about 200 °C to about 450 °C, preferably from about 200 °C to about 300 °C.

5. The method (100) according to any one of the previous claims, wherein at step a) the polyurethane A comprises at least two polyurethanes.

6. The method (100) according to any one of the previous claims, wherein at step f) the isocyanate E is separated from the alcohol F by distilling away the alcohol F at a temperature from about 100 °C to about 300 °C, preferably from about 200 °C to about 250 °C, more preferably from 210 °C to about 230 °C.

7. The method (100) according to any one of the previous claims, wherein at step c) the alcoholysis reaction is performed at a temperature equal to or lower than the boiling point of alcohol B.

8. The method (100) according to claim 7, wherein at step c) the alcoholysis reaction is performed at a temperature from about 120 °C to about 240 °C, preferably from about 150 °C to about 200 °C, more preferably from 180 °C to about 190 °C.

9. The method (100) according to any one of the previous claims, wherein the polyurethane A provided at step a) is a polyurethane made from MDI, and at step e) the thermolysis reaction is performed on a dicarbamate of MDI at a temperature from 230 °C to about 280 °C, preferably from 240 °C to 270 °C, more preferably around 250 °C.

10. The method (100) according to any one of claims 3 to 9, wherein at d2) the second alcohol G is a C₁-C₁₅ alcohol, preferably a C₁-C₁₀ alcohol, more preferably a C₄-C₈ alcohol.

11. The method (100) according to any one of the previous claims, wherein at step b) the first alcohol B is selected from the list comprising: propanol, pentanol, isopropyl alcohol, butanol, hexanol, nonanol, octanol, glycerol, ethylene glycol, diethylene glycol, triethylene glycol.

12. The method (100) according to any one of the previous claims, wherein at step d2) the second alcohol G is selected from the list comprising: propanol, pentanol, isopropyl alcohol, butanol, hexanol, nonanol, octanol, glycerol, ethylene glycol, diethylene glycol, triethylene glycol.

13. The method (100) according to any one of the previous claims, wherein at step b) and/or d2) the first alcohol B and/or the second alcohol G is provided in excess, wherein the molar excess of said first alcohol B and/or second alcohol G with respect to the polyol D is at least 2:1, preferably exceeding 3:1, more preferably exceeding 4:1.

14. The method (100) according to any one of the previous claims, wherein at step e) the thermolysis reaction (103) is performed at a pressure from about 1 bara (100 kPa) to about 10 mbara (1 kPa), preferably from about 1 bara (100 KPa) to about 100 mbara (10 kPa) more preferably from about 1 bara (10 kPa) to about 200 mbara, even more preferably from 1 bara (10 kPa) to about 0.9 bara (90 kPa).

15. The method (100) according to any one of claims 1 to 13, wherein at step e) the thermolysis reaction (103) is performed under an inert gas.

## Patentansprüche

1. Verfahren (100) zum Recyceln eines Polyurethans, umfassend die Schritte:
a) Bereitstellen eines Polyurethans A;
b) Bereitstellen eines ersten Alkohols B, entweder polyfunktionell, oder monofunktionell der aus der Liste ausgewählt ist, umfassend: Propanol, Pentanol, Isopropylalkohol, Butanol, Hexanol, Nonanol, Octanol;
c) Durchführen einer Alkoholysereaktion (101) des Polyurethans A, das in Schritt a) mit dem ersten Alkohol B bereitgestellt wird, das in Schritt b) bereitgestellt wird, wobei dadurch ein Carbamat C und ein Polyol D erhalten werden;
d) Abtrennen (102) des Carbamats C von dem Polyol D, das in Schritt c) erhalten wird, wobei dadurch das Carbamat C bereitgestellt wird;
e) Durchführen einer Thermolysereaktion (103) an dem Carbamat, das in Schritt d) bereitgestellt wird, wobei dadurch ein Isocyanat E und ein Alkohol F erhalten werden, wobei der Alkohol F ein Produkt der Thermolysereaktion an dem Carbamat ist, das in Schritt d) bereitgestellt wird; und
f) Abtrennen (104) des Isocyanats E von dem Alkohol F.

2. Verfahren (100) nach Anspruch 1, wobei in Schritt b) der erste Alkohol B Ethylenglycol ist.

3. Verfahren (100) nach einem der vorstehenden Ansprüche, wobei Schritt d) ferner die Schritte umfasst:
d2) Bereitstellen eines zweiten Alkohols G, entweder mono- oder polyfunktional, der sich von dem ersten Alkohol B unterscheidet, das in Schritt b) bereitgestellt wird;
d3) Durchführen einer Umcarbamylierungsreaktion (201) an dem Carbamat C, das in Schritt d) bereitgestellt wird, wobei dadurch ein Carbamat H aus dem zweiten Alkohol G, das in Schritt d2) bereitgestellt wird, und dem ersten Alkohol B erhalten wird;
d4) Abtrennen (202) des Carbamats H, das in Schritt d3) erhalten wird, von dem ersten Alkohol B.

4. Verfahren (100) nach einem der vorstehenden Ansprüche, wobei in Schritt e) die Thermolysereaktion (103) bei einer Temperatur von etwa 200 °C bis etwa 450 °C, vorzugsweise von etwa 200 °C bis etwa 300 °C durchgeführt wird.

5. Verfahren (100) nach einem der vorstehenden Ansprüche, wobei in Schritt a) das Polyurethan A mindestens zwei Polyurethane umfasst.

6. Verfahren (100) nach einem der vorstehenden Ansprüche, wobei in Schritt f) das Isocyanat E von dem Alkohol F durch Abdestillieren des Alkohols F bei einer Temperatur von etwa 100 °C bis etwa 300 °C, vorzugsweise von etwa 200 °C bis etwa 250 °C, mehr bevorzugt von 210 °C bis etwa 230 °C abgetrennt wird.

7. Verfahren (100) nach einem der vorstehenden Ansprüche, wobei in Schritt c) die Alkoholysereaktion bei einer Temperatur durchgeführt wird, die gleich oder niedriger als der Siedepunkt des Alkohols B ist.

8. Verfahren (100) nach Anspruch 7, wobei in Schritt c) die Alkoholysereaktion bei einer Temperatur von etwa 120 °C bis etwa 240 °C, vorzugsweise von etwa 150 °C bis etwa 200 °C, mehr bevorzugt von 180 °C bis etwa 190 °C durchgeführt wird.

9. Verfahren (100) nach einem der vorstehenden Ansprüche, wobei das Polyurethan A, das in Schritt a) bereitgestellt wird, ein Polyurethan ist, das aus MDI hergestellt ist, und in Schritt e) die Thermolysereaktion an einem Dicarbamat von MDI bei einer Temperatur von 230 °C bis etwa 280 °C, vorzugsweise von 240 °C bis 270 °C, mehr bevorzugt bei etwa 250 °C durchgeführt wird.

10. Verfahren (100) nach einem der Ansprüche 3 bis 9, wobei bei d2) der zweite Alkohol G ein C₁-C₁₅-Alkohol, vorzugsweise ein C₁-C₁₀-Alkohol, mehr bevorzugt ein C₄-C₈-Alkohol ist.

11. Verfahren (100) nach einem der vorstehenden Ansprüche, wobei in Schritt b) der erste Alkohol B aus der Liste ausgewählt wird, umfassend: Propanol, Pentanol, Isopropylalkohol, Butanol, Hexanol, Nonanol, Octanol, Glycerin, Ethylenglycol, Diethylenglycol, Triethylenglycol.

12. Verfahren (100) nach einem der vorstehenden Ansprüche, wobei in Schritt d2) der zweite Alkohol G aus der Liste ausgewählt wird, umfassend: Propanol, Pentanol, Isopropylalkohol, Butanol, Hexanol, Nonanol, Octanol, Glycerin, Ethylenglycol, Diethylenglycol, Triethylenglycol.

13. Verfahren (100) nach einem der vorstehenden Ansprüche, wobei in Schritt b) und/oder d2) der erste Alkohol B und/oder der zweite Alkohol G im Überschuss bereitgestellt wird, wobei der molare Überschuss des ersten Alkohols B und/oder des zweiten Alkohols G hinsichtlich des Polyols D mindestens 2 : 1 beträgt, vorzugsweise 3 : 1 überschreitet, mehr bevorzugt 4 : 1 überschreitet.

14. Verfahren (100) nach einem der vorstehenden Ansprüche, wobei in Schritt e) die Thermolysereaktion (103) bei einem Druck von etwa 1 bara (100 kPa) bis etwa 10 mbara (1 kPa), vorzugsweise von etwa 1 bara (100 kPa) bis etwa 100 mbara (10 kPa), mehr bevorzugt von etwa 1 bara (10 kPa) bis etwa 200 mbara, noch mehr bevorzugt von 1 bara (10 kPa) bis etwa 0,9 bara (90 kPa) durchgeführt wird.

15. Verfahren (100) nach einem der Ansprüche 1 bis 13, wobei in Schritt e) die Thermolysereaktion (103) unter einem Inertgas durchgeführt wird.

## Revendications

1. Procédé (100) de recyclage d'un polyuréthane, comprenant les étapes consistant à :
a) fournir un polyuréthane A ;
b) fournir un premier alcool B, soit polyfonctionnel, soit monofonctionnel choisi dans la liste comprenant : propanol, pentanol, alcool isopropylique, butanol, hexanol, nonanol, octanol ;
c) mettre en œuvre une réaction d'alcoolyse (101) du polyuréthane A fourni à l'étape a) avec le premier alcool B fourni à l'étape b), ce qui permet d'obtenir un carbamate C et un polyol D ;
d) séparer (102) le carbamate C du polyol D, obtenu à l'étape c), en fournissant de ce fait le carbamate C ;
e) mettre en œuvre une réaction de thermolyse (103) sur le carbamate fourni à l'étape d), ce qui permet d'obtenir un isocyanate E et un alcool F, cet alcool F étant un produit de ladite réaction de thermolyse sur le carbamate fourni à l'étape d) ; et
f) séparer (104) l'isocyanate E de l'alcool F.

2. Procédé (100) selon la revendication 1, dans lequel à l'étape b) le premier alcool B est de l'éthylène glycol.

3. Procédé (100) selon l'une quelconque des revendications précédentes, dans lequel l'étape d) comprend en outre les étapes :
d2) fourniture d'un second alcool G, soit mono- soit poly-fonctionnel, différent du premier alcool B fourni à l'étape b) ;
d3) réalisation d'une réaction de transcarbamation (201) sur le carbamate C fourni à l'étape d), ce qui permet d'obtenir un carbamate H du second alcool G fourni à l'étape d2), et le premier alcool B ;
d4) séparer (202) le carbamate H obtenu à l'étape d3) du premier alcool B.

4. Procédé (100) selon l'une quelconque des revendications précédentes, dans lequel à l'étape e) la réaction de thermolyse (103) est mise en œuvre à une température allant d'environ 200 °C à environ 450 °C, de préférence d'environ 200 °C à environ 300 °C.

5. Procédé (100) selon l'une quelconque des revendications précédentes, dans lequel à l'étape a) le polyuréthane A comprend au moins deux polyuréthanes.

6. Procédé (100) selon l'une quelconque des revendications précédentes, dans lequel à l'étape f) l'isocyanate E est séparé de l'alcool F en éliminant par distillation l'alcool F à une température allant d'environ 100 °C à environ 300 °C, de préférence d'environ 200 °C à environ 250 °C, plus préférablement de 210 °C à environ 230 °C.

7. Procédé (100) selon l'une quelconque des revendications précédentes, dans lequel à l'étape c) la réaction d'alcoolyse est mise en œuvre à une température égale ou inférieure au point d'ébullition de l'alcool B.

8. Procédé (100) selon la revendication 7, dans lequel à l'étape c) la réaction d'alcoolyse est mise en œuvre à une température allant d'environ 120 °C à environ 240 °C, de préférence d'environ 150 °C à environ 200 °C, plus préférablement de 180 °C à environ 190 °C.

9. Procédé (100) selon l'une quelconque des revendications précédentes, dans lequel le polyuréthane A fourni à l'étape a) est un polyuréthane fabriqué à partir de MDI, et à l'étape e) la réaction de thermolyse est mise en œuvre sur un dicarbamate de MDI à une température allant de 230 °C à environ 280 °C, de préférence de 240 °C à 270 °C, plus préférablement à environ 250 °C.

10. Procédé (100) selon l'une quelconque des revendications 3 à 9, dans lequel en d2) le second alcool G est un alcool en C₁ à C₁₅, de préférence un alcool en C₁ à C₁₀, plus préférablement un alcool en C₄ à C₈.

11. Procédé (100) selon l'une quelconque des revendications précédentes, dans lequel à l'étape b) le premier alcool B est choisi dans la liste comprenant : propanol, pentanol, alcool isopropylique, butanol, hexanol, nonanol, octanol, glycérol, éthylène glycol, diéthylène glycol, triéthylène glycol.

12. Procédé (100) selon l'une quelconque des revendications précédentes, dans lequel à l'étape d2) le second alcool G est choisi dans la liste comprenant : propanol, pentanol, alcool isopropylique, butanol, hexanol, nonanol, octanol, glycérol, éthylène glycol, diéthylène glycol, triéthylène glycol.

13. Procédé (100) selon l'une quelconque des revendications précédentes, dans lequel à l'étape b) et/ou d2) le premier alcool B et/ou le second alcool G est fourni en excès, dans lequel l'excès molaire dudit premier alcool B et/ou second alcool G par rapport au polyol D vaut au moins 2:1, de préférence dépassant 3:1, plus préférablement dépassant 4:1.

14. Procédé (100) selon l'une quelconque des revendications précédentes, dans lequel à l'étape e) la réaction de thermolyse (103) est mise en œuvre à une pression d'environ 1 bar absolu (100 kPa) à environ 10 mbar absolus (1 kPa), de préférence d'environ 1 bar absolu (100 kPa) à environ 100 mbar absolus (10 kPa) plus préférablement d'environ 1 bar absolu (10 kPa) à environ 200 mbar absolus, même plus préférablement de 1 bar absolu (10 kPa) à environ 0,9 bar absolu (90 kPa).

15. Procédé (100) selon l'une quelconque des revendications 1 à 13, dans lequel à l'étape e) la réaction de thermolyse (103) est mise en œuvre sous un gaz inerte.
